(19)

Europäisches Patentamt
European Patent Office
Office européen des brevets

(11) **EP 4 508 976 A1**

(12) **EUROPEAN PATENT APPLICATION**

(43) Date of publication:
**19.02.2025 Bulletin 2025/08**

(21) Application number: **23192016.6**

(22) Date of filing: **17.08.2023**

(51) International Patent Classification (IPC):
*A01H 1/04* (2006.01)   *A01K 67/02* (2006.01)

(52) Cooperative Patent Classification (CPC):
**A01H 1/04;** G16B 20/40

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Wageningen Universiteit**
  **6708 PB Wageningen (NL)**

• **Stichting Wageningen Research**
  **6708 PB Wageningen (NL)**

(72) Inventors:
• **CALUS, Mario Pieter Lea**
  **6708 PB Wageningen (NL)**
• **NIEHOFF, Tobias Alexander Maria**
  **6708 PB Wageningen (NL)**

(74) Representative: **Nederlandsch Octrooibureau**
  **P.O. Box 29720**
  **2502 LS The Hague (NL)**

(54) **METHOD FOR SELECTING INDIVIDUALS AND/OR MATINGS IN A BREEDING POPULATION**

(57) The present invention provides a method for identifying parent individuals and/or matings in a breeding population that are expected to produce better performing offspring in the grandoffspring generation or a further, later generation. The method identifies individuals based on a genetic selection criterion that is at least dependent on an expected breeding value, BV, of a progeny individual from the grand offspring generation or a further generation. Also provided is a computer-readable medium comprising instructions for performing the method.

Fig. 1A

**Description**

**Technical Field**

The invention relates to a method for selecting individuals and/or matings in a breeding population, for example to optimize genetic gain when the selected individuals and/or matings would be used in a breeding program. Furthermore, the invention relates to a computer readable medium comprising instructions to perform the method.

**Background Art**

Breeding programs are typically used to increase the average genetic level of one or more subsequent generations of a defined population in comparison to the current generation. Here the expected genetic level of a generation is defined as the average performance of that generation with respect to a certain phenotypic trait of interest, for example the milk production of cows or the grain yield of soybeans. The difference in average genetic level between two subsequent generations is referred to as genetic gain. Typically, a group of individuals is selected from the current generation and mated with partners from the selected group of individuals, to optimize genetic gain for the next generation and thereby maximally improve the genetic level of future generation(s).

The selection of individuals typically takes place on the basis of a genetic selection criterion. Several such criteria exist, yet the one that has traditionally been used most is the Genomic Estimated Breeding Value (GEBV). Individuals are ranked based on their GEBV, and the best ranked animals are selected to serve the market needs and/or as parents for breeding the next generation. This selection method is here referred to as "truncation selection based on breeding values". The assignment of breeding pairs, also referred to as matings, may be done based on the genetic selection criterion. Alternatively, the assignment may be done based on a different criterion, for instance to control the amount of inbreeding, or the assignment may be done randomly.

Disadvantageously, the individuals that best serve the market needs may not necessarily produce the best performing offspring with the highest genetic level. For example, if two parents are selected whose favorable alleles are largely overlapping, the future improvement of the genotype of their offspring will be limited to obtaining fixed homozygous allele states for these loci, and missing the selection opportunity to gather additional favorable alleles on alternative loci by introduction via other parents. Therefore other methods have been proposed.

Nowadays, the implicit primary goal of most commercial breeding programs is focused on a different approach, wherein the aim lies on selecting parents that do not necessarily need to have high performance themselves, but should instead have the ability to produce offspring with a very high genetic level. Other genetic selection criteria have therefore been defined, which are typically dependent on the GEBV and the gametic Mendelian Sampling Variance (gametic MSV) of the parent individual.

Although criteria that take the gametic MSV into account have been shown to improve the genetic level of future generations in comparison to truncation selection based on breeding values, further improvements may still be made. It is a goal of the present invention to formulate better genetic selection criteria that can be used to further improve on methods for selecting and/or mating individuals. Alternatively, or in addition, it is a goal of the invention to maintain sufficient biodiversity across generations while producing the best possible offspring.

**Definitions**

In the following description and examples, a number of terms are used. In order to provide a clear and consistent understanding of the description and claims, including the scope to be given such terms, the following definitions are provided for the terms as used in the description and claims. Unless otherwise defined herein, all technical and scientific terms used have the same meaning as commonly understood by one of ordinary skill in the art to which this invention belongs.

Selection and selection criteria: process, model, system or algorithm in order to choose one or more individuals in a population that will contribute genetic material to the next generation. In particular, such a process, model, system or algorithm can be based both on natural or artificial phenomena or procedural steps. Selection criteria can be based on phenotypic or genomic characteristics, for instance, but not limited to, the presence, or degree of presence, of genes, gene expression, genetic markers, combinations of genes, quantitative trait loci, traits or combinations of traits.

Breeding value (BV): the genetic merit of an individual to a breeding program as determined by the individual's contribution to at least one phenotypic trait of interest.

Estimated breeding value (EBV): an approximation of an individual's breeding value, in particular based on the estimated difference between the average performance of that individual's offspring and the average performance of all offspring in a randomly mating population. The estimated average performance of all offspring in a randomly mating population may take into account that individuals with inter-familial relationships, i.e. pedigree relations, normally do not mate.

Expected breeding value (ExBV): The breeding value of an individual in a future generation is a random variable. The expected breeding value of the offspring of a particular mating may be computed as an average of the BVs of all possible offspring or fullsibs of that mating or the average of the breeding values of the parents.

Genomic estimated breeding value (GEBV): estimated breeding value based on genome-wide information, i.e. information derived from different or remote (genetic) loci of the genome such as loci of different chromosomes. In particular, genomic estimated breeding values are an approximation of an individual's genomic genetic merit, determined by the contribution to at least one phenotypic trait of interest of an individual's genome-wide genes or genome-wide (genetic) loci, or genome-wide haplotypes or genome-wide molecular marker scores in a breeding program aimed at improving the at least one phenotypic trait of interest.

Gametes: Reproductive cells of an individual comprising sperm and egg cells produced by the individual.

Gametic Mendelian sampling variance (gametic MSV): the variance of BV's among the gametes that are produced by an individual.

Estimated gametic Mendelian sampling variance (estimated gametic MSV): an approximation of the gametic MSV based on the estimated breeding value (EBV) or genomic estimated breeding value (GEBV).

Expected gametic Mendelian sampling variance (expected gametic MSV): the gametic MSV of an individual in a future generation is a random variable. The expected gametic MSV of the offspring of a particular mating may be computed as an average of the gametic MSV of all possible offspring or fullsibs of that mating.

Mendelian Sampling Variance (MSV): the variance of the GEBV among fullsibs of a mating.

Genetic variance: the variance of breeding values of all individuals in a group of individuals, for instance including all individuals of a single generation.

Usefulness Criterion (UC): an approximation of the expected performance among the top offspring of a mating, e.g., 5 % of best offspring of a mating, or 10% of best offspring of a mating.

Genetic level: Average phenotypic performance of a group of individuals.

Conventional genetic gain: The conventional genetic gain of the (N+1)st generation is a population-wide performance indicator and relates to the increase in average GEBV from the Nth to the (N+1)st generation, where N is any natural number.

Commercial genetic gain: The commercial genetic gain of the (N+1)st generation is a subpopulation performance indicator and relates to the increase in average GEBV from the Nth to the (N+1)st generation of a selected group of top individuals, where N is any natural number. For example, the average BV among the top 10% best performing individuals, i.e. the top 10% with the best BV.

Individual: a living subject, in particular a (non-human) animal or (crop) plant.

Parent: the term "parent" or "parent individual", refers to the selected individuals of the breeding population, or zeroth generation, that are selected for mating and producing the individuals of the offspring generation.

Offspring: the term "offspring" or "offspring individual", refers to a first generation individual obtained by intercrossing two parent individuals.

Grand-offspring: the term "grand offspring" or "grand offspring individual", refers to a second generation individual obtained by intercrossing two individuals from the offspring generation.

Phenotype: the composite of an individual's characteristics or traits, particularly observable characteristics or traits, such as, but not limited to morphological, physical, biochemical, developmental or behavioral characteristics or traits. The phenotype of an individual can be formed by the expression of genes as well as environmental factors, and on interactions between gene expression and environmental factors.

Phenotypic trait of interest: a heritable characteristic of a plant or animal species which may be quantified in a certain unit of measure. Examples of quantitative phenotypic traits of interest are (but are not limited to) for plants: fruit size, fruit count, yield in kg per ha, plant height, relative growth speed, flowering time, germination rate, leave area, disease resistances, yield components, biochemical composition, and for animals: milk yield, milk protein content, carcass weight, fodder conversion, body fat composition, litter size, coat color, resistance to diseases. It can be desired that a quantitative phenotypic trait of interest is increased or decreased, and the respective shift of the average value for the characteristic in the population can improve the economic value of that population, variety or offspring relative to the parent generation(s).

Genotype: as used herein, the term "genotype" refers to the genetic makeup of a cell, an organism, or an individual (i.e. the specific allele makeup of the individual) usually with reference to a specific character or phenotypic trait of interest under consideration. However, not all organisms with the same genotype necessarily look or act the same way because appearance and behavior are modified by environmental and developmental conditions. Likewise, not all organisms that look alike necessarily have the same genotype.

Genotype/phenotype relationship model: a model that can associate (correlate) genotype with phenotype for individuals in a population. To create such a model it is typically required to phenotype individuals of a population and genotype the same individuals. In particular, genotyping can be based on high-density marker data, such as data on the presence or absence of a SNP at a plurality of loci. Likewise, phenotyping can be performed, for example by measuring the value for the quantitative phenotypic trait of interest per individual. The genotype/phenotype relationship model can then be created by calculating correlations between the genotypic data and the phenotypic data. For example, with dense marker arrays, such as SNP arrays, some markers can be correlated with positive or negative effects on a particular quantitative phenotypic trait of interest. In this way, the model can attribute a contribution to the quantitative phenotypic trait of interest to the presence or absence of a marker. Said contribution may for example be expressed in kg, m, L, depending on the unit of measure as used for the quantitative phenotypic trait of interest (for example fruit size, milk production, etc.).

Locus: as used herein, the term "locus" or "loci" (plural) refers to a specific site (place) or sites on the genome. For example, the "locus" refers to the site in the genome where the two alleles of the locus are found (for diploid organisms).

Quantitative trait loci (QTLs): sites on the genome containing alleles that are associated to a quantitative trait (based on the genotype/phenotype relationship model).

Allele: the term "allele" refers to the nucleotide sequence variant that is observed at a particular locus. A diploid individual has two positions for one allele per locus, one position on either one of the two homologous chromosomes. For each of the positions of a particular locus, one or more alternative nucleotide sequence variants may exist in a population, i.e. for each position different possible alleles may exist in a population. However, each individual can have only one of the possible alleles on each one of the positions of a locus. The alternative nucleotide sequence variants, i.e. the different possible alleles, differ at least slightly in nucleotide sequence, and typically can be distinguished based on the presence or absence of at least one SNP or InDel. When referred herein to an "allelic state", reference is made to the presence or absence of an allele at a position within a particular locus, which can be expressed as the presence or absence of the respective marker (e.g. SNP or indel) at the particular locus.

Attributed Allele substitution effect: this term refers to the estimated quantitative effect on the trait when for a given locus one allele (e.g. as measured by presence of a particular SNP) is substituted by another allele under consideration (e.g. as measured by absence of the particular SNP) within a given genetic and/or environmental background. For example, if fruit yield is the quantitative phenotypic trait of interest in a population of plants, the quantitative effect on that trait may be expressed in kg. Based on the genotype/phenotype relationship model, a particular allele on a given locus (e.g. as measured by presence of a particular SNP) can thus be attributed an allele substitution effect of e.g. 0.0001 kg, which means that if the particular allele is replaced by the other possible allele (e.g. as measured by absence of the particular SNP), the quantitative effect on the trait, i.e. fruit yield is estimated to be 0.0001 kg.

Heterozygous and homozygous: as used herein, the term "heterozygous" refers to a genetic condition existing when two different alleles reside at a specific locus, for example a locus having alleles A/B, wherein A and B are positioned individually on either of the two homologous chromosomes. Conversely, as used herein, the term "homozygous" refers to a genetic condition existing when two identical alleles reside at a specific locus, for example a locus having alleles A/A, positioned individually on each of the two homologous chromosomes.

**Summary of the Invention**

According to a first aspect of the invention, there is provided a method for identifying parent individuals and/or matings in a breeding population. The method comprises selecting a plurality of parent individuals in the breeding population available for mating, or a plurality of potential matings that involve available parent individuals; determining a value of a genetic selection criterion for each of the plurality of selected parent individuals or of the plurality of selected potential matings, wherein the genetic selection criterion is at least dependent on an expected breeding value, BV, of a progeny individual from a grand offspring generation or a further generation, the progeny individual having the parent individual or the parent individuals involved in the potential mating as ancestor(s); and identifying the one or more parent individuals and/or matings that have the best value for the genetic selection criterion.

In this context, the term breeding value (BV) is used to refer to an estimated breeding value (EBV), a genomic estimated breeding value (GEBV) or another variable directly related or correlating with one of those. For example, a predicted transmitting ability (PTA) of a gamete, which is known to correspond to half of the breeding value, i.e., BV/2, is also considered to be included in the term BV.

Advantageously, the genetic selection criterion that is used for the identification of the individuals or matings in a breeding population in the present invention looks ahead further than the parent generation and offspring generation. The present inventors came to the realization that by considering the long-term impact on the breeding process when making a selection, the genetic gain and/or a level of gametic MSV in the offspring generation, grand-offspring generation and further generations could be improved.

It was found that by using information on the performance of individuals from the grand-offspring generation or further, more genetic variance could be preserved for future generations. This is particular advantageous as it increases the likelihood that top performing individuals are created in further generations, and in addition positively influences the population's biodiversity. It thereby may also mitigate problems associated with inbreeding and may allow for breeding top individuals in further generations.

An example of a selection or identification problem is provided to explain the principle underlying the present disclosure. Table 1 provides an illustrative example of a very small population of three diploid individuals. Gender is neglected in this simplified example. The genotypes of the individuals have been established, and are represented by three loci indicated at the top side of the table. If an allele is marked with a "+", a positive contribution is provided to a desired trait value according to a genomic prediction, whereas a "-" indicates all other possible alleles. The best achievable genotype is the one that contains the highest fraction of "+" alleles.

Table 1:

|  | Loci 1 | Loci 2 | Loci 3 | BV |
|---|---|---|---|---|
| Individual A | - | - | - | 2 |
|  | + | + | - |  |
| Individual B | - | - | - | 2 |
|  | + | + | - |  |
| Individual C | - | - | + | 2 |
|  | - | - | + |  |

A Breading Value (BV) can be assigned to each individual, awarding each positive contribution "+" with a value of 1, whereas negative contributions "-" receive the value zero. Hence all three individuals in this example would have the same BV of 2, and therefore the expected performance of a random offspring individual is the same for the matings M1: AxB, M2: AxC or M3: BxC. The expected performance may be expressed in terms of the expected BV, which is 2 for each mating.

Following the prior art selection procedure with a genetic selection criterion solely based on the BV, e.g., truncation selection based on BVs, the individuals with the highest BV would have been selected as parents for the next generation. As the individuals in Table 1 all have the same BV, this means that the selection would have been made randomly. Any individual could be identified best on the genetic selection criterion as a best performing individual.

Nevertheless, if the goal is to produce individuals with a very high BV in the offspring generation, then mating M1 should be preferred because the offspring of this mating show a higher variance in their breeding values than the offspring of matings M2 and M3. This is because individuals A and B have a higher gametic variance than individual C. In other words, it is more likely to obtain offspring with a high BV from the mating M1 than from the mating M2 or M3. In particular, offspring of the mating M1 could potentially obtain a BV of 4, whereas offspring of the matings M2 and M3 can never obtain a BV higher than 3.

If the focus was however on the grand offspring generation, then matings M2 and M3 are to be preferred because the offspring of these matings show higher gametic MSV and thus increase the chance of selecting a very good individual. In other words, there is a chance to produce gametes with a breeding value of 3 from offspring of mating M2 and M3 whereas the probability for gametes with a breeding values of 3 of offspring of the mating M1 is 0. By looking ahead more generations, better individuals may be produced in such further generations.

It will be understood by the skilled person that an expected BV of individuals of the grand offspring generation or a further generation depends on the BV of their ancestors in the earlier generations such as the offspring generation and the parent generation. In addition, the gametic MSV of the ancestors may have an influence on the likelihood that progeny of a parent individual or mating of parent individuals turns out to be a top performing individual.

In an embodiment, the genetic selection criterion is further at least dependent on an expected gametic Mendelian Sampling Variance, MSV, of the same or another progeny individual from the offspring generation and/or a later generation. Selecting individuals with a better expected gametic Mendelian Sampling Variance for mating may improve the likelihood of producing very good or exceptionally good individuals.

In an embodiment, the identified parent individuals and/or matings have, as compared to at least 50% of the other parent individuals and/or matings within the breeding population, a better expected breeding value of the progeny individual for at least one phenotypic trait of interest, and/or a higher expected genetic level of progeny in the grand offspring generation or a further generation, and/or a larger expected probability for their progeny individuals of the grand offspring generation or a further generation to be selected for market needs and/or for breeding a next generation.. Alternatively, the comparison may be done with respect to at least 60% or at least 70% or at least 80% or at least 90% of all other parent individuals and/or matings in the breeding population. Advantageously, the selection method is aimed at obtaining high performing individuals in the grand offspring generation or further generations.

In this context, we refer to a "better" BV to indicate the favorable character of the trait. In literature, this is typically referred to as a higher BV, yet it will be understood that dependent on the definition, a lower BV may sometimes also be favourable. Here the terms better BV and higher BV are used interchangeably to indicate the most favorable BV. In addition, we refer to an expected value as it should be considered that the BV of offspring tracing back to an individual or a potential mating of the breeding population is a random variable. The value therefore cannot be found or estimated based on the performance of the individual, but should be assessed as the expected value among all offspring produced by a certain individual, or resulting from a certain mating.

As per its definition, the expected BV of an individual from a certain generation provides information on the performance of an individual from that generation. The expected gametic MSV of an individual, however, is considered to provide information on the variance of expected BV of its own offspring. For example, the gametic MSV of an offspring individual provides information on the genetic level of an individual from the grand-offspring generation. Likewise, a selection criterion that is dependent on the gametic variance of parent individuals is considered to comprise information on the performance of the offspring generation. A higher expected gametic MSV of an individual increases the probability to produce high performing offspring themselves.

It will be understood by the skilled person that in principle any expression for the expected performance of later generation individuals such as offspring individuals or grandoffspring individuals may be expressed in terms of the BV and gametic MSV of ancestor individuals, possibly accompanied by parameters of the breeding program such as the selection intensity. Nevertheless, the genetic selection criterion according to the invention distinguishes itself from existing criteria by using a combination of said variables that is physically representative for the expected performance of progeny individuals from the grand offspring generation or further, later generations.

The expected BV and expected gametic MSV of an individual from the offspring generation are just one of several parameters that can be used to provide information on the performance of the grand-offspring generation or further generation. The genetic selection criterion may also be dependent on other parameters in addition.

In an embodiment, the genetic selection criterion is dependent on an expected variance of a breeding value of the grand-offspring produced by selected offspring that traces back to an individual or mating of the current selection. A higher variance of a generation or of fullsibs in a generation increases the probability of producing top ranked individuals in later generations that optimally serve the market needs. In addition, biodiversity may be better preserved.

In a further embodiment, the genetic selection criterion is aimed at optimizing an expected conventional genetic gain of a target generation or an expected commercial genetic gain of a target generation, wherein the target generation is the grand offspring generation or a further generation. Specifically, such population-wide performance indicators may be used when the selection method is a computer-implemented method solved using an optimization solver. The genetic gain may be considered as a measure to define the overall increase in the average genetic level of the population for conventional genetic gain, or the average genetic level of a top part of the population for commercial genetic gain. For example, the commercial gain may be defined as the genetic gain achieved for the top 10% of the population, or the top 20% of the population, or the top 30% of the population. The genetic gain is typically expressed in terms of BVs or genetic levels, yet may also be defined differently.

In an embodiment, the genetic selection criterion is aimed at optimizing an expected genetic level of selected individuals in a target generation, wherein the target generation is the grand-offspring generation or a further generation. Here the genetic level may be considered as a measure of the overall, average performance of the selected individuals in the grand-offspring generation or the further generation..

In an embodiment, the genetic selection criterion is dependent on genetic performance across at least three different generations of the breeding program. For example, the genetic selection criterion may be based on the genetic performance of individuals in the breeding population, the offspring generation, and the grand-offspring generation. By making the criterion dependent on multiple generations, it may be prevented that there are generations without individuals that perform well.

In an embodiment, the genetic selection criterion is further dependent on one or more of the following: a breeding value of one or more individuals of the breeding population, a gametic MSV of one or more individuals of the breeding population; and/or a selection intensity for a selection step in any generation in any generation of a breeding program. Such parameters may have an effect on the selection procedure. For instance, if the selection intensity is relatively high, only a few parents are selected and therefore those parents should have a high gametic MSV as otherwise as otherwise the variance in the offspring generation may be very low. On the contrary, with a lower selection intensity the gametic MSV is less important as offspring individuals trace back to a larger number of parents.. The selection intensity may thus have an effect on whether an individual with relatively low gametic MSV may be selected or not.

In an embodiment parent individuals are identified, wherein the method further comprises assigning matings within the population of identified parent individuals. Such assignment may take place randomly or using a mating algorithm. In an embodiment, the mating algorithm is dependent on the value of the genetic selection criterion. This allows to select the most promising matings.

In an embodiment, a plurality of potential matings are selected and the value of the genetic selection criterion is computed for each of the plurality of potential matings. This can be advantageous as even the best individual of a breeding population, e.g., the individual with the highest BV or genetic level, should not be selected if no suitable mating partner can be found within the breeding population.

According to an important embodiment of the invention, the genetic selection criterion is based on a probability that the grand offspring of the potential mating has a BV above a certain pre-determined threshold. Hence the genetic selection criterion is aimed at selecting those individuals for a mating that would lead to the production of the best individuals in the grand-offspring generation. Such individuals can be selected to serve the market needs in the grand-offspring generation and/or for breeding of the subsequent generation. In an embodiment, the threshold is defined based on the market needs. In an alternative embodiment, the threshold is defined such that a certain percentage of top performing animals is selected, for example the top 10% or the top 20% or the top 30% of the individuals from that generation.

According to another important embodiment of the invention, the genetic selection criterion is based on the expected breeding value of selected grand offspring of the potential mating. Here the term "selected" grand-offspring implies that a certain selection needs to be carried out. In an embodiment, such grand-offspring is assumed to be selected based on having a BV above a certain pre-determined threshold. This threshold may be determined in absolute terms and in relation with a quantitative trait of interest. Alternatively, in an embodiment, such grand-offspring is selected based on the selection intensity, e.g., by selecting a top percentage of the generation individuals regardless of their actual genetic performance.

In an embodiment, the calculation of the genetic selection criterion is performed based on the evaluation of an analytical expression. Advantageously, the method is computationally cheap in comparison to methods that simulate one or more realizations of the breeding program. Moreover, analytic expressions provide insight into the underlying principles of the selection criterion, which may make it easier to explain to breeders. In particular, the evaluation of an analytical expression is here considered as an alternative to selection methods using a Monte Carlo Simulation type of approach wherein a large number of realizations of a breeding program can be simulated and its results afterwards be used for the approximation of genetic performance indicators such as the expected BV or expected gametic MSV that traces back to a certain individual or mating.

In an embodiment, the step of identifying one or more individuals and/or matings includes: ranking the parent individuals and/or the matings based on the highest estimated value of the genetic selection criterion, and applying a truncation selection to select a top portion of the parent individuals and/or the matings. An absolute number of individuals, a percentage, or all individuals with the genetic selection criterion above a certain threshold may be identified. This may also be dependent on the selection intensity and/or the number of matings that an individual in the breeding population may be involved in.

In an embodiment, the top 5% or the top 10% or the top 15% is selected, optionally wherein some of the parent individuals and/or the matings are selected more than once.

In an embodiment, the method further comprises assigning a plurality of matings among the identified parent individuals dependent on the value of the genetic selection criterion. For example, potential matings may be ranked based on the highest estimated value of the genetic selection criterion. Using truncation selection, a plurality of matings may be selected for breeding.

In an embodiment, all individuals of the breeding population are selected in the selection step. Alternatively, one or more individuals may be deselect. A deselection may be done on the basis of the same or another genetic selection criterion. For example, in an embodiment truncation selection based on BV is carried out to eliminate the worst performing individuals from the breeding population. Thereafter, the genetic selection criterion according to an embodiment of the invention is applied to select the individuals and/or matings. The underlying rationale is that the worst performing individuals would never be eligible for breeding as they cannot contribute enough positive traits to the next generation. In embodiments, up to the worst 10% or up to the worst 20% or up to the worst 30% or more individuals may be deselected in a pre-selection step.

In an embodiment, the individuals are animals, preferably livestock.

In an embodiment, the population of individuals is of a species selected from the group consisting of Cattle (*Bos taurus, Bos indicus*), Water buffalo (*Bubalus bubalis*), Equine (*Equus caballus*), Sheep (*Ovis aries*), Goat (*Capra hircus*), Pig (*Sus scrota*), Chicken (*Gallus gallus*), Turkey (*Maleagris gallopavo*), Ducks (*Anas platyrhynchos, Cairina moschata*), Geese (*Anser anser domesticus, Anser cygnoides*), Pigeons (*Columba livia domestica*), Rat (*Rattus novergicus*), Mouse (*Mus musculus*), Cat (*Felis catus*), Dog (*Canis familiaris*), Rabbit (*Oryctolagus cuniculus*), Guinea pig (*Cavia porcellus*), Zebra fish (*Danio rerio*) and Fruit fly (*Drosophila melanogaster*).

In an embodiment, the population of individuals is of insects, for example black soldier flies (Hermetia illucens) or Mealworm (Tenebrio molitor).

In another preferred embodiment, the population of individuals is of a fish species selected from the group consisting of *Cyprinus carpio, Salmo salar, Oreochromis niloticus, Oncorhynchus mykiss, Ctenopharyngodon idella, Hypophthalmichthys molitrix, Gibelion catla, Cyprinus carpio, Hypophthalmichthys nobilis, Carassius carassius, Oreochromis niloticus, Pangasius pangasius and Labeo rohita, or wherein the method is applied to a shrimp species selected from the group consisting of Macrobrachium rosenbergii, Litopenaeus vannamei and Penaeus monodon.*

Alternatively, the population of individuals may be a field crop, or vegetable crop, or woody fruit species, or forestry species, or plantation crop, preferably selected from the group consisting of Arabidopsis thaliana, Abyssinian mustard, alfalfa, barley, barrel clover, black mustard, buckwheat, canola, clover, common flax, common vetch, corn spurry, coffee, cotton, Egyptian clover, fodder beet, hemp, hop, Indian mustard, Jerusalem artichoke, maize, millet, mustard, lupin, oat, oilseed rape (Brassica napus), field mustard (Brassica rapa), opium poppy, Persian clover, potato, red clover, rye, safflower, sisal, soy bean, sugar beet, sunflower, tea, tobacco, triticale, wheat, white clover, white mustard, wild rice, winter vetch, artichoke, asparagus, asparagus beans, aubergine, beetroot, black radish, black bean, black salsify, broad bean, broccoli, Brussels sprouts, cabbage, cantaloupe, carrot, cauliflower, celery, chard, chicory, chili pepper, chinese cabbage, choi sum, common bean, corn salad, courgette, cucumber, daikon, eggplant, endive, fennel, garlic, goosefoot, green bean, Indian lettuce, kale, kidney bean, kohlrabi, leek, lettuce, lentil, lima bean, maize, melon, mizuna, napa cabbage, onion, parsnip, pea, pepper, potato, pumpkin, quinoa, radicchio, radish, rapini, red cabbage, rhubarb, runner bean, rutabaga, salad rocket, Savoy cabbage, shallot, soy bean, spinach, squash, sugar cane, swede, tomatillo, tomato, turnip, watercress, watermelon, yellow turnip, almond, apple, apricot, bird cherry, butternut, cashew, cherry, chokeberry, crabapple, filbert, greengage, hawthorn, hazel, heartnut, loquat, medlar, mirabelle prune, nectarine, peach, peacherine, pear, pecan, pistacio, plum, prune, quince, rowan, walnut, acacia, alder, Allegheny chinkapin, American beech, American chestnut, American hornbeam, ash, aspen, basswood, beech, bigtoothed, aspen, birch, bitternut hickory, black alder, black birch, black cherry, black gum, black locust, black maple, black oak, black poplar, black walnut, black willow, butternut, cedar, chestnut, chestnut oak, Chinese chestnut, Corsican pine, cottonwood, crabapple, cucumbertree, cypress, dogwood, Douglas fir, Eastern hemlock, elm, English oak, eucalyptus, European beech, European larch, European silver fir, European white birch, fir, flowering dogwood, gum, hawthorn, hornbeam, horse chestnut, hybrid poplar, Japanese chestnut, Japanese larch, larch, lodgepole pine, maple, maritime pine, mockernut hickory, Norway spruce, oak, Oregon pine, Pacific silver fir, pedunculate oak, pignut hickory, pine, pitch pine, poplar, Scots pine, sweet chestnut, red alder, red cedar, red maple, red oak, red pine, red spruce, redwood, rowan, sassafrass, Scots pine, Serbian spruce, serviceberry, shagbark hickory, silver birch, Sitka spruce, southern beech, spruce, striped maple, sugar maple, sweet birch, sweet chestnut, sycamore, tamarack, tulip tree, Western hemlock, white ash, white oak, white pine, yellow birch, banana, breadfruit, coconut, date palm, jackfruit, mango, oil palm, olive, papaya, pineapple, plantain, rubber tree and sugar palm.

In an embodiment, the breeding value is the genomic estimated breeding value (GEBV), and the method comprises determining the GEBV of each individual in the breeding population.

In a further embodiment, the method comprises a) providing a genotype/phenotype relationship model, wherein the model estimates for a given genotype of an individual what the quantitative contribution is of the allele substitution effects of said plurality of loci on at least one phenotypic trait of interest; and b) genotyping each individual within the breeding population, preferably by collecting genotypic data for each individual within the breeding population; c) calculating within the breeding population the allele substitution effect for each allele of said plurality of loci by using the genotype/phenotype relationship model; and d) determining the GEBV of each individual in the breeding population.

In a further embodiment, the method comprises step e) computing the estimated gametic MSV of each individual in the breeding population. This may be done according to conventional methods based on allele substitution effects, phased genotype data and expected recombination frequencies between loci.

In an embodiment, the step of providing a genotype/phenotype relationship model comprises: a) providing a training population of individuals; b) collecting phenotypic data for at least one trait of interest for each individual within the training population; and c) collecting genotypic data for each individual within the breeding population using molecular marker techniques, sequence-based genotyping or whole genome sequencing, and attributing to each allele of a plurality of loci of each individual, an allele substitution effect for the at least one phenotypic trait of interest; and d) deriving the genotype/phenotype relationship model for the training population of individuals.

In an embodiment, the method is a computer-implemented method, or at least partly a computer-implemented method. In a further embodiment, at least the steps of determing the genetic selection criterion and identifying the individuals are computer-implemented. In yet a further embodiment, the assignment of matings is a computer-implemented step.

In an embodiment, the steps of identifying individuals based on the genetic selection criterium and assigning matings is carried out subsequently. Alternatively, these steps may be carried out simultaneously as part of an optimization problem.

According to a second aspect of the invention, and in accordance with the advantages as described herein above, there is provided a computer-readable medium comprising instructions for performing the method according to the invention. A computer, based on the instructions, may output a selection of individuals from the breeding population, or a selection of matings based on the breeding population.

In an embodiment, the instructions further include a method for assigning a mating to each individual from the group of selected individuals. Such a method, also referred to as a mating algorithm, may be based on the same or on another genetic selection criterion.

It will further be understood by the skilled person that planning two or more generations ahead does not mean that the resulting breeding program needs to be carried out exactly according to plan. After a new generation of individuals has been produced, i.e., the offspring generation, new data becomes available. This data provides valuable information for formulating the next step in the breeding program and is typically used for the selection of individuals and/or matings for breeding the generation thereafter, i.e., the grand offspring generation. Hence although the selection method takes further generations into account, this may be performed only theoretically or in simulations. Typically a new selection procedure will be carried out to select individuals and/or matings in each step of a breeding program.

Not included in the scope of protection are methods involving crossing and subsequent selection of plants or animals or other essentially biological processes in the sense of Article 53(b) EPC.

According to another aspect of the invention, and in accordance with the advantages as described herein above, there is provided a method for producing offspring from a breeding population comprising selecting individuals for mating using the genetic selection criterion according to the invention, and producing offspring from the selected parent individuals and/or the selected matings. In a further embodiment, the method may further comprise producing grand-offspring from the produced offspring of the selected parent individuals and/or the selected matings.

**Brief Description of Drawings**

Embodiments will now be described, by way of example only, with reference to the accompanying schematic drawings in which corresponding reference symbols indicate corresponding parts.

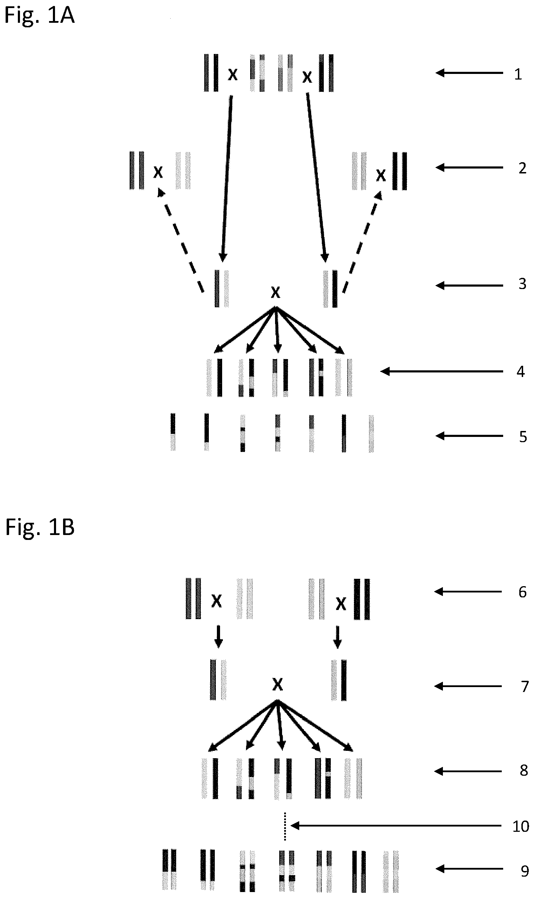

Figure 1 provides a graphical representation of a method for computing the expected gametic Mendelian sampling variance of an offspring individual according to a first embodiment.

The figures are meant for illustrative purposes only, and do not serve as restriction of the scope or the protection as laid down by the claims.

**Description of Embodiments**

The following is a description of certain embodiments of the invention, given by way of example only and with reference to the figures.

*Prior art genetic selection criteria*

For illustrating the advantages of the method, some existing genetic selection criteria are briefly discussed first. Although each of these criteria can be found in literature, they are included here for the sake of completeness. The criteria are used later on to demonstrate the performance of the method and genetic selection criterion according to the invention.

**BV:** Selection on BV is done by truncation selection. Here $BV_{animal}$ is considered, which is the breeding value of an individual, here assumed to be an animal, without considering its possible mates or its gametic MSV. It will be understood that although the examples are provided for animals, the application of the invention to other individuals such as plant species is not excluded.

**ProbSelOff:** This criterion is associated with the probability that the offspring of a mating is selected for further breeding of the subsequent generation. The probability of a mating is defined as the fraction of offspring that is expected to have a higher breeding value than the selection threshold. This may be computed by Equation 1 as:

$$ProbSelOff = 1 - \Phi\left(\frac{\tau_{p_O} - \left(\frac{BV_{sire} + BV_{dam}}{2}\right)}{\sqrt{\sigma^2_{FS_{mating}}}}\right). \qquad (1)$$

Here $\Phi$ is the cumulative normal distribution function with $\tau_{p_O}$ the absolute truncation selection point in the offspring generation based on selected proportion $p$ of the population, i.e., $\tau_{p_O} = \overline{BV}_{pop} + \left(i_p + x_p\right)\sqrt{\sigma^2_{BV}}$, where $\overline{BV}_{pop}$ is the average breeding value of the population in the current generation, $i_p$ is the selection intensity belonging to selected proportion $p$ of the population, $x_p$ is the normalized truncation selection point belonging to selected proportion $p$ of the population and $\sigma^2_{BV}$ is the variance of breeding values in the population in the current generation. The term ($\frac{BV_{sire} + BV_{dam}}{2}$) is the average breeding value of full sib offspring. Finally, $\sigma^2_{FS_{mating}}$ is the variance of breeding values of full sib offspring of a mating, given as the sum of the gametic MSV of the sire and dam involved in the mating. That is,

$$\sigma^2_{FS_{mating}} = \sigma^2_{gamMS_{sire}} + \sigma^2_{gamMS_{dam}} \qquad (2)$$

Basing selection decisions on the probability of a mating to produce offspring that show a higher breeding value than the truncation selection point has been proposed in animal breeding by Segelke et al. (2014).

**ExpBVSelOff:** This criterion is associated with the expected breeding value of the selected fraction of offspring of a mating. In literature, it has also been referred to as the Usefulness criterion (Schnell and Utz, 1975). The expected breeding value of the selected fraction of offspring of a mating, is the average breeding value of the top ranked offspring individuals. Hence this criterion is again dependent on the selection intensity of the population. The expected breeding value of selected offspring may be calculated from Equation 3:

$$ExpBVSelOff = \left(\frac{BV_{sire}+BV_{dam}}{2}\right) + i_p * \sqrt{\sigma^2_{FS_{mating}}} \qquad (3)$$

Here the variables and parameters are the same as defined before in relation to ProbSelOff.

Index5: This index represents the linearized probability of an individual to produce top-ranking offspring. It is the linearization of ProbSelOff, assuming a randomly assigned mate. The calculation of the index value may be done using Equation 4:

$$I_5 = BV_{animal} + x_p * \sqrt{2 * \sigma^2_{gamMS_{animal}}} \qquad (4)$$

Here $BV_{animal}$ and $\sigma^2_{gamMS_{animal}}$ refer to a single animal of the population. $x_p$ is the normalized truncation selection point belonging to selected proportion $p$ of the population. Hence the Index5 is a selection criterion that does not take into account a possible mate of the individual and is instead dependent on only the individual itself.

Each of the above described selection criteria depend on the BV and the gametic MSV of individuals from the parent generation (N=0). In addition, the criteria ExpBVSelOff is defined as an expected breeding value of selected offspring, and therefore it is considered that this criterion also depends on the expected breeding value of at least an individual from the offspring generation (N=1). There is no dependency of the criteria on the genetic level of later generation individuals, i.e., individuals of generation (N≥2). Here "N" is used to refer to the generation number, with N=0 for the parent generation, N=1 for the offspring generation, N=2 for the grand offspring generation, and so forth for further, later generations where N≥3. Genetic selection criteria according to embodiments of the invention, however, do comprise information on the genetic performance of individuals from generations later or further than the offspring generation, i.e., for generations N≥2.

According to a first embodiment of the invention, a genetic selection criterion is used that is dependent on the expected BV of the offspring, the variance of BV of the fullsib offspring of a mating, and the gametic MSV of the offspring of a mating. Beneficially, the genetic selection criterion depends on performance indicators for the grand-offspring generation (N=2), which prevents that animals are selected that would produce offspring that is very good itself, but would be unlikely to produce good grand-offspring. An example of a criterion based on these genetic performance indicators is ProbSelGr-Off.

**ProbSelGrOff:** This genetic selection criterion is based on the estimated Probability to Select Grand-Offspring (ProbSelGrOff) of a certain mating to be selected for a breeding themselves. Herein it is assumed that the grand-offspring would be selected based on traditional truncation selection based on breeding values. In other words, matings are selected based on the probability that a breeding value of their grandoffspring would be higher than the predefined absolute truncation selection threshold that should be applied to the grandoffspring generation.

An expression for this criterion may be derived by considering that the selection of grand offspring is conditional on the selection of their parents from the offspring generation, i.e.,

$$ProbSelGrOff = ProbSelOff * P(P_{GO}|selected\ offspring), \qquad (5)$$

where *ProbSelOff* is the probability that offspring of a mating will be selected as described in Equation 1 above, and where

$$P(P_{GO}|selected\ offspring) = 1 - \Phi\left(\frac{\tau_{p_{GO}} - \left(\frac{\overline{BV}_{sel\ off\ mating} + \overline{BV}_{sel\ ind\ pop}}{2}\right)}{\sqrt{\sigma^2_{gam_{sel\ off\ mating}} + \sigma^2_{gam_{sel\ ind\ pop}}}}\right). \qquad (6)$$

Here it has been assumed that individuals from the offspring generation that have been selected are randomly mated to other selected individuals from the same generation.

Advantageously, this criterion combines an existing criterion for the selection of parents to obtain good offspring, with a new criterion to obtain also good grand-offspring. Consequently, individuals that would produce very bad offspring are excluded from the selection as ProbSelOff would be very small thereby resulting also in ProbSelGrOff to be very small. This genetic selection criterion thereby provides a good balance of accounting for the performance of later generations while simultaneously preventing that very bad offspring is produced. The latter is also important as typically every generation needs to provide both individuals that can serve the market needs, as well as individuals that can parent the further generation.

Nevertheless, ProbSelOff may also be small for different reasons than a low BV of the parent individuals. For example, if two parents both have a low gametic MSV, e.g. because they are highly inbred, this would also lead to a low probability to select their offspring. If the parents are unrelated and carry different beneficial haplotypes, however, their offspring could have a good performance with a high gametic MSV. Hence the grand-offspring that could be produced from such a mating, could have a good enough performance for the individual to be selected. However, even with a high conditional probability that grand offspring would be selected, the overall probability that the grand offspring would be selected is close to zero due to the low probability that its parents from the offspring generation are selected, i.e., a low ProbSelOff. Alternative genetic selection criteria may be used to overcome this drawback.

**ExpBVSelGrOff:** According to another embodiment of the invention, the genetic selection criterion is based on the Expected Breeding Value in Selected Grand offspring (ExpBVSelGrOff). The criterion ExpBVSelGrOff corresponds to the expected genetic level of selected grand offspring that are produced by selected offspring of a mating.

An expression for this criterion can derived as:

$$ExpBVSelGrOff = \frac{\frac{BV_{sire} + BV_{dam}}{2} + i_O * \sigma_{FS} + \overline{BV}_{t+0} + i_{p_{t+0}} * \sigma_{BV_{t+0}} + i_{p_{t+1}} * \sigma_{BV_{t+1}}}{2}$$
$$+ i_{GO} * \sqrt{\frac{(1-k_O)*\sigma_{FS}^2}{4} + \frac{(1-k_{p_{t+1}})*\sigma_{BV_{t+1}}^2}{4} + \bar{\sigma}_{gamMS_1\,off}^2 + \bar{\sigma}_{gamMS_1\,ind}^2}. \qquad (7)$$

Here the variable t indicates the generation number of the initial breeding population. $\bar{\sigma}_{gamMS_1\,ind}^2$ is the average gametic MSV of an individual in the population in the offspring generation. No method for accurate calculation is known, therefore the average gametic MSV of all individuals in the parent generation can be used as an approximation instead. The term $k$ is the variance reduction coefficient and can be calculated based on the selection intensity i and the normalized truncation selection point $x$, i.e., $k = i * (i - x)$.

The selection intensities are as follows: $i_{p_{t+0}}$ is the selection intensity that is to be applied to the individuals of the population in the current generation, $i_{p_{t+1}}$ is the selection intensity that is to be applied to the individuals of the population in the next generation, $i_O$ is the selection intensity that is to be applied to the offspring, and $i_{GO}$ is the selection intensity that is to be applied to the offspring. In an embodiment, a single selection intensity may be used for all four parameters. Advantageously, this can significantly simplify the criterion.

It will be understood by the skilled person that it is not needed to use the full expression and actually evaluate the expected breeding value to rank the animal. It typically suffices to evaluate only those terms of the criterion that vary dependent on the selected parent individuals. For example, evaluation of the average breeding value of individuals from the breeding population (N=0) may be omitted without changing the results.

In addition, under certain assumptions, the above expression may be simplified to provide certain derived criteria. In an embodiment, the adapted criterion in Equation 8 is used:

$$\widetilde{ExpBVSelGrOFF} = 0.25 * (BV_{sire} + BV_{dam}) + i_p * \left(0.5 * \sqrt{\sigma_{FS_{mating}}^2} + \sqrt{\sigma_{GO}^2}\right) \qquad (8)$$

Here all constants are removed and it is further assumed that the same selection intensity $i_p$ is used across generations, and that the variance in BV remains substantially the same over generations. It will be understood, however, that this are assumptions for the derivation of the criterion. Also when the selection intensity is not constant, or when the genetic variance varies considerably, the genetic selection criterion may still be applied.

It will be understood by the skilled person that this genetic selection criterion, and any other genetic selection criterion as discussed herein above, may be adjusted by the skilled person to be based on the estimated probability to select offspring from an arbitrary Nth generation, typically wherein N is larger than 2 or equal to 2. Here N=0 is the breeding population, N=1 the offspring generation, N=2 the grand-offspring generation and so forth.

For example, for ProbSelGrOff, the probability that individuals in the third generation are selected, can be considered conditional to the probability that their parents in the second generation have been selected. More in general, the probability that individuals in the Nth generation are selected, can be considered conditional to the probability that their parents in the (N-1)st generation have been selected. By assuming that the individuals from any generation between the offspring generation up to and including the (N-1)st generation have been randomly mated to other selected individuals of their own generation, further genetic selection criteria for values of N>2 can be derived analogously as was done here for N=2.

*Calculation of gametic Mendelian sampling variance for selected offspring of a mating*

The calculation of the genetic selection criteria according to the invention may require an estimation of the gametic Mendelian sampling variance of the selected offspring of a mating, i.e., $\sigma^2_{gam_{sel\,off\,mating}}$. As the present inventors are not aware that this has been approximated in literature previously, an exemplary method of how to obtain an approximation is presented below.

In an embodiment, the approach of Allier, Moreau, et al. (2019) may be followed. They presented an analytical method to calculate the variance of double haploid lines created from four fully homozygous founder lines in a plant breeding setting. Double haploids are individuals that are derived by doubling the chromosomes of a gamete of an individual to restore diploidy and produce fully homozygous lines. Double haploids produced by four fully inbred lines are conceptually comparable to gametes produced by fullsibs (full siblings) in an animal breeding setting. Figure 1 visualizes this similarity.

Fig. 1A illustrates the transmittance of chromosome segments in an animal breeding setting. Figure 1B shows a double haploid population based on four inbred founders considered by the approach of Allier, Moreau, et al. (2019). As Fig. 1A illustrates, the chromosomes of the true grandparents 1 of a plurality of fullsibs 4 are replaced by the pseudo chromosomes 2 of pseudo grandparents. The pseudo chromosomes are passed on to the future generations including the parents 3, fullsibs 4, and the gametes 5 of fullsibs for even further generations. By pretending that the grandparents in an animal breeding setting are fully homozygous for the chromosome that was inherited by the parent, these "pseudo grandparents" in animal breeding are identical to the actual inbred founder lines 6 considered in the approach by Allier, Moreau, et al. (2019). As a comparison between Fig. 1A and 1B illustrates, the parents 3 have the same chromosomes as the two-way F1 parents 7; the fullsibs 4 have the same chromosomes as the four-way F1 parents 8, and the gametes 5 are the same as the double haploid individuals 9, yet showing only one chromosome instead of two. The double haploid individuals are obtained after double haploid induction 10.

The gametic MSV of offspring of a particular mating can be computed as follows:

$$\bar{\sigma}^2_{gamMS_{1\,off}} = \frac{\left(\sigma^2_{DH} - \left(\sigma^2_{gametes_{sire}} + \sigma^2_{gametes_{dam}}\right)\right)}{4} = \frac{(\sigma^2_{DH} - \sigma^2_{FS})}{4} \qquad (8)$$

This formulation has been mathematically derived, wherein the correctness of the formulation has been proven in simulations. The variance of the double haploids, $\sigma^2_{DH}$, was computed with the analytical equation presented by Allier, Moreau, et al. (2019) for four-way DH populations. For the calculation of $\sigma^2_{gametes_{sire}}$ and $\sigma^2_{gametes_{dam}}$, the method presented by Musa and Reinsch (2023) was used.

It will be understood by the skilled person that this is just one method to evaluate the gametic MSV of offspring individuals. It will further be understood by the skilled person how to find expressions for the gametic MSV of grand offspring generation and/or further generations.

*Experimental*

In validation simulation experiments, the criteria **ExpBVSelGrOff** and **ProbSelGrOff,** i.e., the criteria that look two generations ahead, were compared with four existing genetic selection criteria: **ExpBVSelOff; ProbSelOff; Index5** and **BV.** For each method, the realized progress through simulated breeding and selection was determined and compared. The setup and results of the validation simulation experiments are now explained step-by-step. All code for this study was run in R version 4.1.2.

All methods use the same starting material for the simulations. A nucleus breeding population with recurrent selection was simulated using the simulation software MoBPS version 1.10.45.

The model animal used in the simulations contained 30 chromosomes each with a length of 100 cM. It was assumed that the true QTL effects, the genetic map and the haplotypes of all model animals is known. A randomly generated (parental) starting population of size 5000 model animals was generated, comprising 2500 males and 2500 females. In total 3000 generations of random mating were simulated to establish a linkage disequilibrium among markers. The population size was set to decrease linearly to 50 animals in generation 2997, and then increased linearly to 5000 animals in generation 3000.

After simulating the population history, about 15,000 markers of initially 63,000 were still segregating. 3000 purely additive QTL effects were placed on randomly selected markers from a pool of markers with a minor allele frequency above 0.05. The QTL effects were drawn from a gamma distribution with a shape parameter of 0.4. These settings resulted in a coefficient of variation of the standard deviation of gametic BV of 0.095 in generation 0.

To reduce genetic variance due to the Bulmer effect, an additional burn-in phase of 5 generations, i.e., generation 3001 to 3005, was simulated with selection based on BVs and random mating among selected individuals. The number of animals in generations 3001 to 3005 was kept at 5000 per generation.

The breeding program was started at generation 3006 and continued for 21 generations until generation 3026. The population size in the breeding program was 4000 per generation. In every generation, 20 females and 20 males were selected based on one of the genetic selection criteria. Each of the selected parents contributed equally to the next generation, having 200 offspring each.

For ExpBVSelOff, ProbSelOff, ProbSelGrOff and ExpBVSelGrOff, the following selection algorithm was applied:

1) Rank all individuals based on the genetic selection criterion for all potential mating partners based on the value calculated for the respective genetic selection criterion.
2) The highest rank an individual ever got from the opposite sex is noted
3) The individual with the lowest highest rank (i.e. the "least liked" animal) is removed from the list of selection candidates
4) Repeat step 1 until the desired number of males and females are selected.

Hence the genetic selection criterion is determined for each potential mating, whereas steps 2 and 3 are used to select individuals rather than matings. It will be understood that in alternative embodiments, also matings may be selected directly based on the ranking.

After selecting individuals, matings need to be assigned. Mating was restricted so that no more than 40 offspring individuals are produced from any one mating. This ensured that every individual is mated to at least 25% of available partners of the opposite sex.

For selection based on BV and Index5, mating was performed randomly. A mate allocation algorithm was used when selection was based on the ExpBVSelOff, ProbSelOff, ExpBVSelGrOff and ProbSelGrOff - criterion. The mate allocation algorithm was developed with the aim to have a chance for every animal to be mated to every selected animal of the opposite sex.

1) Sample a female randomly
2) If the female has not reached the number of offspring it should produce, continue. Otherwise, go to step 1.

   a. Pick the male that is most preferred by the female
   b. If the male has not yet reached its number of offspring it should produce, continue. Otherwise, go to step 2a to pick the next best male.
   c. If the maximum number of offspring allowed for this mating has not yet been reached, continue. Otherwise, go to step 2a to pick the next best male.

3) Mate the selected female with the chosen male.
4) Go back to step 1 until the required number of matings has been achieved.

The algorithm requires to pick one of the sexes to draw from in step 1, and then use the other in step 2. This choice is unlikely to be reciprocal, i.e. the best matching male to mate with a particular female may not have this same female as its best matching mate. To account for these non-reciprocal choices, for half the number of offspring, i.e., 2000 individuals, the mating partners for the females were chosen. For the other half, the mating partners for the males were chosen.

In order to reduce computation time, 500 individuals (25%) of all selection candidates were preselected per sex based on their BV. Bijma et al. (2020) show in their Figure 2 that a preselection intensity as stringent as 10% does not have a negative effect when using Index5 values. Because selection was not only made based on Index5 values, 25% of all animals were preselected based on their BV. For these preselected candidates, the Index5 values, ExpBVSelOff and ProbSelOff were calculated and subsequently used for selection. In scenarios in which selection was based on the ProbSelGrOff and ExpBVSelGrOff criteria, a second preselection step was used in which four times more animals than needed for replacement were preselected from each sex based on their Index5 values (16% of all BV preselected candidates).

The simulation and subsequent analyses were repeated 100 times to produce reliable estimates on the method comparison.

*Results*

Table 2 shows the results of the comparison and the conventional genetic gain and the commercial genetic gain that is achieved using each of the selection criteria. Although the conventional genetic gain in the first generation is slightly less in comparison to the results achieved using the criteria that only account for the short term, on the long term this is recovered and the proposed methods wherein the criterion is based on looking more than one generation ahead, is optimized. The commercial genetic gain, was at no point compromised.

Table 2:

| | Conventional genetic gain in generation | | | Commercial genetic gain in generation | | |
|---|---|---|---|---|---|---|
| | 1 | 5 | 21 | 1 | 5 | 21 |
| BV | 2.66 | 11.92 | 32.53 | 5.17 | 13.90 | 33.24 |
| ExpBVSelOff | 2.58 | 12.08 | 33.59 | 5.21 | 14.23 | 34.36 |
| ProbSelOff | 2.59 | 12.06 | 33.59 | 5.20 | 14.22 | 34.38 |
| Index5 | 2.60 | 12.13 | 33.44 | 5.21 | 14.25 | 34.18 |
| ExpBVSelGrOff | 2.47 | 12.18 | 34.01 | 5.20 | 14.55 | 34.99 |
| ProbSelGrOff | 2.54 | 12.18 | 34.05 | 5.23 | 14.46 | 34.96 |

An important advantage of the method according to the present invention is the better preservation of the useful diversity to improve the trait across generations. Table 3 shows the genetic standard deviation after 1, 5, and 21 generations, and the genic standard deviation after 1, 5, and 21 generations. The genetic standard deviation was obtained by taking all selection candidates in a certain generation, mating them randomly without selection and continue doing so for three more generations to get rid of the variance reducing effect of the selection methods. In other words, the genetic variance obtained after 4 generations of intercrossing without selection reflects the genetic variance in an unselected base population and should thus not be biased to either of the selection methods or criteria. The values are expressed as a percentage relative to the starting population, i.e., zeroth generation, of the breeding program. In the starting population, the standard deviations are 100% and calculations follow the style of $100\% \left( \frac{\sqrt{varG_{criterion\_gen21}}}{\sqrt{varG_{crterion\_gen0}}} \right)$. Table 3 confirms that all selection criteria preserve the variance better than the traditional method based on BV.

Table 3:

| | Genetic standard deviation in generation | | | Genic standard deviation in generation | | |
|---|---|---|---|---|---|---|
| | 1 | 5 | 21 | 1 | 5 | 21 |
| BV | 95.01 | 75.6 | 27.1 | 95.86 | 79.1 | 31.8 |
| ExpBVSelOff | 97.20 | 79.4 | 28.4 | 96.54 | 80.4 | 31.5 |
| ProbSelOff | 97.21 | 79.7 | 29.3 | 96.52 | 80.5 | 32.1 |
| Index5 | 97.13 | 78.4 | 27.8 | 96.45 | 80.0 | 31.6 |
| ExpBVSelGrOff | 99.83 | 84.6 | 36.5 | 97.24 | 81.8 | 33.7 |
| ProbSelGrOff | 98.39 | 81.3 | 32.9 | 96.87 | 81.1 | 32.9 |

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. It will be apparent to the person skilled in the art that alternative and equivalent embodiments of the invention can be conceived and reduced to practice. All changes which come within the meaning and range of equivalency of the claims are to be embraced within their scope.

For instance, the application of the method to a model animal should not be considered limited to objects that only classify as animals. Instead, other individuals such as plants would also be possible. In particular, the individuals can be of different ploidy nature. For example, the population of individuals can be of a diploid, allopolyploid, or autopolyploid species.

Furthermore, it will be understood by the skilled person that the ranking step, selection step, and mating step do not necessarily need to be performed subsequently and independent of each other. For example, a genetic selection criteria according to the invention may also be used in the objective function in an optimization problem, such that ranking, selection and mating of individuals can be done simultaneously. In particular, linear programming may be used to solve such problems.

Examples of the application of the genetic selection criterion and method in an optimization problem is not described in detail. Nevertheless, the skilled person would understand that an objective function may be formulated that takes the performance of individuals from the grand offspring generation or later generations into account. For example, the objective function may depend on one or more of the expected BV and expected gametic MSV of individuals from the offspring generation or later, or depend on the variance in BV among fullsibs of a particular mating. In a first exemplary embodiment, the objective function may be formulated to maximize the sum of expected BVs of all grand offspring individuals. In a second alternative embodiment, the objective function may be formulated to maximize the number of grand offspring individuals that have an expected BV above a certain threshold. The skilled person would further understand how to formulate appropriate constraints to include the specifications of the breeding process. This includes but is not limited to constraints that prescribe the total number of matings that need to be selected in each generation, the maximum number of matings per sire or dame, the maximum number of matings that each sire-dam-combination may maximally produce.

## List of symbols and parameters

$BV_{sire}$　　Breeding value of a sire

$BV_{dam}$　　Breeding value of a dam

$BV_{animal}$　　Breeding value of an animal

$\sigma^2_{gamMS_{sire}}$　　Gametic Mendelian sampling variance of a sire

$\sigma^2_{gamMS_{dam}}$　　Gametic Mendelian sampling variance of a dam

$\sigma^2_{gamMS_{animal}}$　　Gametic Mendelian sampling variance of an animal

$\sigma^2_{BV}$　　Variance of breeding values in the population in the parent generation

$\overline{BV}_{pop}$　　Average breeding value of the population in the parent generation

$i_p$　　Selection intensity belonging to selected proportion $p$ of the population

$x_p$　　Normalized truncation selection point belonging to selected proportion $p$ of the population

$\tau_{pO}$　　Absolute truncation selection point in the offspring generation based on selected proportion $p$ of the population

$$= \overline{BV}_{pop} + \left(i_p + x_p\right)\sqrt{\sigma^2_{BV}}$$

$\tau_{pGO}$　　Absolute truncation selection point in the grand offspring generation based on selected proportion $p$ of the population

$$= \overline{BV}_{pop} + \left(2i_p + x_p\right)\sqrt{\sigma^2_{BV}}$$

$\overline{BV}_{sel\,off\,mating}$　　Expected breeding value of selected offspring of a mating. This is identical to ExpBVSelOff

$\overline{BV}_{sel\,ind\,pop}$　　Average breeding value of individuals of the population 　　$= \overline{BV}_{pop} + i_p\sqrt{\sigma^2_{BV}}$

$\sigma^2_{gam_{sel\,off\,mating}}$　　Variance of breeding values of gametes produced by selected offspring of a particular mating

$\sigma^2_{gam_{sel\,ind\,pop}}{}_{pop}$     Variance of breeding values of gametes produced by selected individuals of the population

$\bar{\sigma}^2_{gamMS_{1\,off}}$     Average gametic Mendelian sampling variance of one random offspring of a mating

$\bar{\sigma}^2_{gamMS_{1\,ind}}$     The average gametic MSV of an individual in the population in the offspring generation.

$\sigma^2_{FS_{mating}}$     Variance of breeding values of full sib offspring of a mating     $\sigma^2_{gamMS_{sire}} + \sigma^2_{gamMS_{dam}}$

$\sigma^2_{GO}$     Variance of grandoffspring produced by selected offspring of a mating when mated to a random partner of selected animals of the population     $=\sigma^2_{gam_{sel\,off\,mating}} + \sigma^2_{gam_{sel\,ind\,pop}}$

**Non-patent literature cited in the description:**

Allier, A., Moreau, L., Charcosset, A., Teyssedre, S., & Lehermeier, C. (2019). Usefulness Criterion and Post-selection Parental Contributions in Multi-parental Crosses: Application to Polygenic Trait Introgression. G3 (Bethesda), 9(5), 1469-1479. https://doi.orq/10.1534/q3.119.400129

Bijma, P., Wientjes, Y. C. J., & Calus, M. P. L. (2020). Breeding Top Genotypes and Accelerating Response to Recurrent Selection by Selecting Parents with Greater Gametic Variance. Genetics, 214(1), 91-107. https://doi.org/10.1534/genetics.119.302643

Musa, A. A., & Reinsch, N. (2023). A similarity matrix for preserving haplotype diversity among parents in genomic selection. bioRxiv, 2023.2006.2001.543227. https://doi.org/10.1101/2023.06.01.543227

Schnell, F., & Utz, H. (1975). Bericht über die Arbeitstagung der Vereinigung österreichischer Pflanzenzüchter. Gumpenstein, Austria: BAL Gumpenstein, 243-248.

Segelke, D., Reinhardt, F., Liu, Z., & Thaller, G. (2014). Prediction of expected genetic variation within groups of offspring for innovative mating schemes. Genet Sel Evol, 46, 42. https://doi.orq/10.1186/1297-9686-46-42

**Claims**

1. A method for identifying parent individuals and/or matings in a breeding population, wherein the method comprises:

   selecting a plurality of parent individuals in the breeding population available for mating, or a plurality of potential matings that involve available parent individuals,
   determining a value of a genetic selection criterion for each of the plurality of selected parent individuals or of the plurality of selected potential matings, wherein the genetic selection criterion is at least dependent on an expected breeding value, BV, of a progeny individual from a grand offspring generation or a further generation, the progeny individual having the parent individual or the parent individuals involved in the potential mating as ancestor(s), and
   identifying the one or more parent individuals and/or matings that have the best value for the genetic selection criterion.

2. The method according to claim 1, wherein the identified parent individuals and/or matings have, as compared to at least 50% of the other parent individuals and/or matings within the breeding population,

   a better expected breeding value of the progeny individual for at least one phenotypic trait of interest, and/or
   a higher expected genetic level of progeny in the grand offspring generation or a further generation, and/or
   a larger expected probability for their progeny individuals of the grand offspring generation or a further generation to be selected for market needs and/or for breeding a next generation.

3. The method according to claim 2, wherein the comparison is done with respect to at least 70%, or at least 90%, of the other parent individuals and/or matings within the breeding population.

4. The method according to any of the preceding claims, wherein the genetic selection criterion is further at least dependent on an expected gametic Mendelian Sampling Variance, MSV, of the same or another progeny individual from the offspring generation, the grand offspring generation, and/or a further generation.

5. The method according to any of the preceding claims, wherein the genetic selection criterion is aimed at optimizing one or more of the following:

   an expected conventional genetic gain of a target generation,
   an expected commercial genetic gain of a target generation, and/or
   an expected average genetic level of selected individuals in a target generation,
   wherein the target generation is the grand offspring generation or a further generation.

6. The method according to any of the preceding claims, wherein the genetic selection criterion is further dependent on one or more of the following:

   a BV of one or more parent individuals of the breeding population,
   a gametic MSV of one or more parent individuals of the breeding population; and/or
   a selection intensity for the selection step in any generation of a breeding program.

7. The method according to any of the preceding claims wherein a plurality of potential matings are selected and wherein the value of the genetic selection criterion is computed for each of the plurality of potential matings.

8. The method according to claim 7 wherein the genetic selection criterion is based on
   a probability that the grand offspring of the potential mating has a BV above a certain pre-determined threshold.

9. The method according to claim 7, wherein the genetic selection criterion is based on the expected breeding value of selected grandoffspring of the potential mating.

10. The method according to any of the preceding claims, wherein the calculation of the genetic selection criterion is performed based on the evaluation of an analytical expression.

11. The method according to any of the preceding claims, wherein the step of identifying one or more individuals and/or matings includes:

   ranking the parent individuals and/or the matings based on the highest estimated value of the genetic selection criterion, and
   applying a truncation selection to select a top portion of the parent individuals and/or the matings.

12. The method according to claim 11, wherein the top 5% or the top 10% or the top 15% is selected, optionally wherein some of the parent individuals and/or the matings are selected more than once.

13. The method according to any of the preceding claims further comprising
   assigning a plurality of matings among the identified parent individuals dependent on the value of the genetic selection criterion.

14. The method according to any of the preceding claims, wherein the individuals are animals, preferably livestock or a species selected from the group consisting of Cattle (*Bos taurus, Bos indicus*), Water buffalo (*Bubalus bubalis*), Equine (Equus caballus), Sheep (Ows aries), Goat (Capra hircus), Pig (*Sus scrofa*), Chicken (*Gallus gallus*), Turkey (*Maleagris gallopavo*), Ducks (*Anas platyrhynchos, Cairina moschata*), Geese (*Anser anser domesticus, Anser cygnoides*), Pigeons (C*olumba livia domestica*), Rat (*Rattus novergicus*), Mouse (*Mus musculus*), Cat (Felis catus), Dog (*Canis familiaris*), Rabbit (*Oryctolagus cuniculus*), Guinea pig (*Caw'a porcellus*), Zebra fish (*Danio rerio*) Fruit fly (*Drosophila melanogaster*).

15. A computer-readable medium comprising instructions for performing the method according to any of the preceding claims.

Fig. 1A

Fig. 1B

Europäisches
Patentamt

European
Patent Office

Office européen
des brevets

## EUROPEAN SEARCH REPORT

**Application Number**

**EP 23 19 2016**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOEINIZADE SABA ET AL: "Optimizing Selection and Mating in Genomic Selection with a Look-Ahead Approach: An Operations Research Framework", G3: GENES, GENOMES, GENETICS, vol. 9, no. 7, 1 July 2019 (2019-07-01), pages 2123-2133, XP093125399, US ISSN: 2160-1836, DOI: 10.1534/g3.118.200842 Retrieved from the Internet: URL:https://academic.oup.com/g3journal/article-pdf/9/7/2123/40634123/g3journal2123.pdf> * Look-ahead selection; page 2125 * * figures 3-5 * * Simulation settings; page 2126 * ----- | 1-13 | INV. A01H1/04 A01K67/02 |
| | -/-- | | |

| | |
|---|---|
| | TECHNICAL FIELDS SEARCHED (IPC) |
| | A01H C12Q G06F A01K C12N G16B |

The present search report has been drawn up for all claims

| Place of search | Date of completion of the search | Examiner |
|---|---|---|
| Munich | 2 February 2024 | Erener Caner, Süheda |

EPO FORM 1503 03.82 (P04C01)

**page 1 of 2**

# EUROPEAN SEARCH REPORT

Europäisches
Patentamt
European
Patent Office
Office européen
des brevets

Application Number

**EP 23 19 2016**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GORJANC GREGOR ET AL: "AlphaMate: a program for optimizing selection, maintenance of diversity and mate allocation in breeding programs", BIOINFORMATICS, vol. 34, no. 19, 1 October 2018 (2018-10-01), pages 3408-3411, XP093125599, GB ISSN: 1367-4803, DOI: 10.1093/bioinformatics/bty375 Retrieved from the Internet: URL:https://academic.oup.com/bioinformatics/article-pdf/34/19/3408/48919396/bioinformatics_34_19_3408.pdf> * abstract * * Materials and methods; page 3409 * * Demonstration; page 3410 * ----- | 1-15 | |
| T | WO 2015/100236 A1 (PIONEER HI BRED INT [US]) 2 July 2015 (2015-07-02) * claim 1 * ----- | | TECHNICAL FIELDS SEARCHED (IPC) |
| T | SANTOS D J A ET AL: "Variance of gametic diversity and its application in selection programs", JOURNAL OF DAIRY SCIENCE, vol. 102, no. 6, 1 June 2019 (2019-06-01), pages 5279-5294, XP085686910, ISSN: 0022-0302, DOI: 10.3168/JDS.2018-15971 * abstract * * table 1 * ----- | | |

The present search report has been drawn up for all claims

| Place of search | Date of completion of the search | Examiner |
|---|---|---|
| Munich | 2 February 2024 | Erener Caner, Süheda |

EPO FORM 1503 03.82 (P04C01)

page 2 of 2

## ANNEX TO THE EUROPEAN SEARCH REPORT
## ON EUROPEAN PATENT APPLICATION NO.

EP 23 19 2016

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2024

| Patent document cited in search report | Publication date | Patent family member(s) | Publication date |
|---|---|---|---|
| WO 2015100236 A1 | 02-07-2015 | AU 2014370029 A1 | 16-06-2016 |
| | | BR 112016015033 A2 | 08-08-2017 |
| | | CA 2932507 A1 | 02-07-2015 |
| | | CN 106028794 A | 12-10-2016 |
| | | EP 3086633 A1 | 02-11-2016 |
| | | PH 12016501255 A1 | 15-08-2016 |
| | | RU 2016130577 A | 01-02-2018 |
| | | US 2016321396 A1 | 03-11-2016 |
| | | WO 2015100236 A1 | 02-07-2015 |
| | | ZA 201603680 B | 29-11-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALLIER, A.** ; **MOREAU, L.** ; **CHARCOSSET, A.** ; **TEYSSEDRE, S.** ; **LEHERMEIER, C.** Usefulness Criterion and Post-selection Parental Contributions in Multi-parental Crosses: Application to Polygenic Trait Introgression. *G3 (Bethesda)*, 2019, vol. 9 (5), 1469-1479, https://doi.orq/10.1534/q3.119.400129
- **BIJMA, P.** ; **WIENTJES, Y. C. J.** ; **CALUS, M. P. L.** Breeding Top Genotypes and Accelerating Response to Recurrent Selection by Selecting Parents with Greater Gametic Variance. *Genetics*, 2020, vol. 214 (1), 91-107, https://doi.org/10.1534/genetics.119.302643

- **MUSA, A. A.** ; **REINSCH, N.** A similarity matrix for preserving haplotype diversity among parents in genomic selection. *bioRxiv*, 2023, https://doi.org/10.1101/2023.06.01.543227
- **SCHNELL, F.** ; **UTZ, H.** Bericht über die Arbeitstagung der Vereinigung österreichischer Pflanzenzüchter. *Gumpenstein, Austria: BAL Gumpenstein*, 1975, 243-248
- **SEGELKE, D.** ; **REINHARDT, F.** ; **LIU, Z.** ; **THALLER, G.** Prediction of expected genetic variation within groups of offspring for innovative mating schemes. *Genet Sel Evol*, 2014, vol. 46, 42, https://doi.orq/10.1186/1297-9686-46-42